(19) Europäisches Patentamt
European Patent Office
Office européen des brevets

(11) EP 2 032 663 B1

(12) **FASCICULE DE BREVET EUROPEEN**

(45) Date de publication et mention de la délivrance du brevet:
**06.05.2015 Bulletin 2015/19**

(21) Numéro de dépôt: **07815059.6**

(22) Date de dépôt: **07.06.2007**

(51) Int Cl.:
***C09D 4/00*** *(2006.01)* ***A61L 27/00*** *(2006.01)*
***C08L 51/10*** *(2006.01)*

(86) Numéro de dépôt international:
**PCT/FR2007/051389**

(87) Numéro de publication internationale:
**WO 2007/141460 (13.12.2007 Gazette 2007/50)**

(54) **PROCÉDÉ DE GREFFAGE DE POLYMÈRES BIOACTIFS SUR DES MATÉRIAUX PROTHÉTIQUES**

VERFAHREN ZUR IMPLANTATION BIOAKTIVER POLYMERE IN PROTHESEN-MATERIALIEN

METHOD FOR GRAFTING BIOACTIVE POLYMERS ON PROSTHETIC MATERIALS

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **07.06.2006 FR 0605073**

(43) Date de publication de la demande:
**11.03.2009 Bulletin 2009/11**

(73) Titulaire: **Université Paris 13**
**93430 Villetaneuse (FR)**

(72) Inventeurs:
- **MIGONNEY, Véronique**
**F-95600 Eaubonne (FR)**
- **HELARY, Gérard**
**F-95640 Santeuil (FR)**
- **NOIRCLERE, Flavie**
**F-92210 Saint-cloud (FR)**

(74) Mandataire: **Tezier Herman, Béatrice et al**
**Cabinet Becker & Associés**
**25, rue Louis le Grand**
**75002 Paris (FR)**

(56) Documents cités:
**EP-A- 0 893 164** **US-A1- 5 782 908**
**US-A1- 5 804 263**

Remarques:
Le dossier contient des informations techniques présentées postérieurement au dépôt de la demande et ne figurant pas dans le présent fascicule.

EP 2 032 663 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne des procédés pour greffer des polymères bioactifs sur un matériau prothétique, les matériaux susceptibles d'être obtenus par ce procédé et des applications de ces derniers. Plus spécifiquement, l'invention concerne un procédé de greffage direct de polymères à la surface de matériaux prothétiques.

L'implantation d'une prothèse dans un site osseux engendre une cascade de réactions tissus/implants intitulée « réponse de l'hôte » qui, si elle est contrôlée, permet d'aboutir in fine à « l'ostéointégration de l'implant ». L'ostéointégration est l'intégration parfaite de l'implant dans l'os grâce à un contact intime tissu osseux/prothèse et à une absence de tissus fibreux à l'interface. Cette intégration est nécessaire pour réhabiliter une fonction déficiente par l'intermédiaire d'implants osseux définitifs (prothèses articulaires, implants dentaires). Ce processus peut cependant être affecté par plusieurs paramètres. D'une part les propriétés physicochimiques du matériau telles que la topographie de surface, la rugosité, la composition chimique et l'énergie de surface, d'autre part la technique chirurgicale et la présence de gennes. De plus, la présence de tissu « fibro-inflammatoire » autour de l'implant, qui n'a ni les activités biologiques, ni les caractéristiques mécaniques du tissu osseux augmente la susceptibilité à un descellement aseptique (désolidarisation de l'implant) et à l'infection du site de l'implantation.

Actuellement, pour assurer l'ancrage de prothèses totales de hanche dans l'os, deux approches sont couramment utilisées : soit l'implant métallique (éventuellement revêtu d'hydroxyapatite) est ancré mécaniquement en force dans l'os, soit il est fixé à l'aide d'un ciment à base de poly (méthacrylate de méthyle) (PMMA). Durant ces trente dernières années, les revêtements « hydroxyapatite » ont été très utilisés pour améliorer l'ostéointégration des implants, car ils permettent un bon ancrage osseux tout en offrant de bonnes performances mécaniques dans un temps relativement court.

Les biomatériaux sont des matériaux destinés à remplacer tout ou partie des fonctions ne pouvant plus être assurées par des tissus, un organe ou une partie d'organe déficients. Pour cela, les biomatériaux implantables doivent principalement répondre à deux critères. Ils doivent d'abord posséder des propriétés mécaniques spécifiques, nécessaires pour assurer correctement les fonctions mécaniques des tissus ou des organes défectueux. Ils doivent aussi ne pas déclencher de réponse hostile de l'hôte c'est-à-dire être « acceptés » et intégrés par l'hôte d'une manière contrôlée, en d'autres termes être « biointégrables ».

Cependant, aucun matériau de synthèse actuellement implanté ne peut réellement être considéré comme « biointégrable ». En effet, le matériau implanté est considéré comme un « corps étranger » par le système vivant et cela déclenche une série d'évènements regroupés sous le terme de réponse inflammatoire, qui si elle n'est pas contrôlée peut alors conduire in fine au rejet de l'implant. Par ailleurs, ce matériau considéré comme « corps étranger » apparaît comme un support privilégié pour l'adhésion et la colonisation de microorganismes tels que les bactéries. L'adhésion de bactéries potentiellement infectantes sur les implants biomédicaux étant l'étape initiatrice de l'infection, représente donc un sérieux problème. D'autre part cette adhésion bactérienne est dans de nombreux cas suivie de la formation d'un « biofilm » qui joue un rôle de protection des bactéries contre toute thérapie et notamment contre les antibiotiques. Par exemple, les infections à staphylocoques suite à l'implantation d'un biomatériau permanent (prothèse orthopédique) ou même temporaire (cathéter) ont souvent des conséquences dramatiques pour les patients comme des septicémies, des endocardites ou des ostéomyélites et leur guérison est extrêmement difficile voire impossible tant que l'implant reste en place. Ainsi, nous observons trop souvent des phénomènes de rejet lors de l'implantation d'un biomatériau dans un organisme vivant ainsi que des phénomènes d'infection des matériaux implantés. Ces réponses biologiques indésirables vis-à-vis de l'implant peuvent conduire à une nouvelle intervention chirurgicale voire même au retrait du biomatériau implanté.

Il convient donc d'optimiser les interactions biomatériaux/cellules dans le cas d'implants articulaires mais également dans le cas des implants dentaires et parallèlement de prévenir l'infection de ces matériaux en contrôlant les interactions biomatériaux/bactéries

Des recherches ont été menées afin d'améliorer la biocompatibilité et la biointégration des matériaux utilisés comme implants et par conséquent diminuer les réponses hostiles de l'hôte conduisant au rejet de l'implant. Un axe de recherche concerne la modification des surfaces des matériaux prothétiques, en particulier la modification de la surface du titane et de ses alliages. Une telle modification a pour but de favoriser la biointégration des implants dans les tissus osseux, tout en prévenant les infections.

Un procédé de greffage de polymères par silanisation a été décrit. Ce procédé permet de fonctionnaliser des surfaces hydroxylées ou riches en $NH_2$ préalablement au greffage d'un polymère. Dans le cas du titane, la silanisation a été principalement utilisée pour créer des interfaces métal-polymère. Les polymères greffés peuvent être des polymères bioactifs, c'est à dire des polymères qui sont capables d'orienter les réponses cellulaires eucaryotes et/ou procaryotes en ce sens que de tels polymères greffés favorisent l'ostéointégration d'un implant prothétique et préviennent le développement d'une infection.

Le protocole de silanisation utilisé sur ces substrats par la plupart des auteurs consiste à attaquer chimiquement la surface de l'échantillon pour favoriser la présence des groupements hydroxyles, puis à immerger l'échantillon dans

une solution contenant la molécule de silane. Les avantages de ces réactions sont leur simplicité et leur stabilité, attribuées à leur liaison covalente et à leur structure en réseau. Toutefois, l'efficacité de la silanisation dépend de la concentration en groupements hydroxyles à la surface du matériau, et cette technique ne permet pas d'immobiliser tous les polymères, mais seulement ceux qui possèdent un groupement fonctionnel compatible avec celui situé en bout de chaîne du dérivé silane.

La plupart des auteurs utilisent donc la silanisation comme une étape intermédiaire au greffage ultérieur d'une molécule bioactive ou organique. Dans la suite de cette demande, ce procédé de greffage par silanisation préalable sera désigné comme «procédé de greffage indirect ».

Les présents inventeurs ont mis au point un nouveau procédé de greffage de polymères bioactifs sur un matériau prothétique.

Un premier aspect de l'invention concerne donc un procédé de greffage de polymères bioactifs sur un matériau prothétique.

Selon un deuxième aspect, l'invention concerne les matériaux prothétiques susceptibles d'être obtenus par un tel procédé.

Selon un dernier aspect, l'invention concerne les implants prothétiques fabriqués à partir des matériaux prothétiques susceptibles d'être obtenus par le procédé de l'invention.

Dans le procédé de greffage de la présente invention, et à la différence du procédé de greffage indirect décrit ci-dessus, aucune molécule intermédiaire, telle qu'un dérivé silane, n'est utilisée entre la surface du matériau et le polymère à greffer dessus.

Ainsi, l'invention a pour objet un procédé de greffage de polymères bioactifs sur un matériau prothétique en titane ou en alliage de titane comprenant les étapes suivantes :

- la génération d'espèces actives donneuses de radicaux libres à la surface du matériau prothétique en titane ou en alliage de titane par oxydation dudit matériau prothétique en titane ou en alliage de titane ;
- la génération de radicaux libres à la surface du matériau prothétique en titane ou en alliage de titane par réaction thermique à une température comprise entre 40 et 10°C.
- la mise en présence dudit matériau prothétique en titane ou en alliage de titane sur lequel des radicaux libres ont été générés avec au moins un monomère porteur d'une fonction permettant une polymérisation radicalaire, la polymérisation radicalaire dudit monomère permettant la formation d'un polymère bioactif, ladite polymérisation radicalaire étan réalisée en l'absence d'oxygène.

Le procédé selon l'invention est un procédé direct, par opposition au procédé indirect décrit ci-dessus, en ce sens que les polymères sont formés directement sur le matériau prothétique à partir des monomères les composant. En effet, dans le présent procédé, des radicaux libres sont formés directement sur le matériau prothétique qui sert alors d'amorceur de la réaction de polymérisation.

Les inventeurs ont pu montrer qu'un tel procédé directe de greffage a plusieurs avantages par rapport au procédé de greffage indirect. Le procédé selon l'invention est notamment plus économique, plus rapide et plus efficace que la méthode indirecte, en particulier en terme de quantité de polymère fixé sur le matériau prothétique. Ainsi, généralement, le procédé de la présente invention permet d'obtenir une densité de greffage 2 à 15 fois supérieure à celle obtenue avec le procédé de greffage indirect par silanisation de l'art antérieur.

Comme spécifié précédemment, le procédé de greffage de la présente invention est exempt de toute étape intermédiaire de greffage d'une molécule intermédiaire entre le matériau prothétique et le polymère greffé.

Au sens de la présente invention, un matériau prothétique est un matériau utilisable dans la fabrication d'un implant médical tel qu'une prothèse, en particulier une prothèse de la hanche ou une prothèse dentaire.

Les matériaux modifiés par le procédé de la présente invention correspondent plus spécifiquement à tout matériau sur la surface duquel des radicaux libres peuvent être formés par oxydation chimique, notamment par oxydation chimique acide ou par ozonation, puis chauffage. A ce titre, on peut notamment citer les supports en métal, incluant le titane et ses alliages, en particulier à base de nickel, de vanadium, d'aluminium et/ou de molybdène, l'aluminium, le tantale, l'iridium, le zirconium, l'or et l'acier, en céramique ou encore en verre. Dans un mode particulier de réalisation de l'invention, le matériau prothétique est un matériau prothétique non polymère.

Avantageusement, le matériau prothétique est le titane ou un de ses alliages.

Le titane est couramment employé comme matériau prothétique car il est biocompatible. En effet, le titane est un matériau de choix pour les implants orthopédiques ou dentaires en raison de ses propriétés mécaniques assez proches de celles de l'os, sa bonne tolérance due à ses capacités d'ostéointégration et/ou à l'absence de phénomène d'allergie ou de rejet vis-à-vis du titane.

Avantageusement, le matériau prothétique peut être poli par abrasion avant formation des radicaux libres pour s'affranchir des problèmes de rugosité de la surface du matériau. Ainsi, dans un mode particulier de réalisation de l'invention, les matériaux prothétiques sont polis avec du papier abrasif et de préférence avec différents papiers abrasifs

de granulométrie décroissante. Plus spécifiquement, le polissage mécanique des matériaux prothétique peut être réalisé à l'aide d'un bras automatique monté sur une polisseuse rotative, avec du papier abrasif de granulométries décroissantes. Ainsi, on peut successivement utiliser des papiers abrasifs de grade 800, 1000 puis 1200. Le matériau prothétique peut être également lavé, de préférence successivement au polissage, notamment dans une solution d'acétone, puis de préférence séché. Il peut être utilisé directement après l'étape de polissage, lavage et/ou séchage, ou peut être conservé en l'absence d'oxygène, de préférence sous atmosphère inerte, telle que sous argon ou hélium.

Avantageusement, les espèces actives donneurs de radicaux libres sont générées à la surface du matériau prothétique par oxydation, en particulier par oxydation chimique. On peut notamment citer une oxydation chimique par l'utilisation d'un mélange acide/$H_2C_2$, en particulier $H_2SO_4/H_2O_2$, ou une oxydation par ozonation. L'oxydation chimique par l'utilisation d'un mélange acide/$H_2O_2$ est particulièrement préférée dans le cas de l'oxydation de matériaux prothétiques métalliques, en particulier l'utilisation d'un mélange $H_2SO_4/H_2O_2$. On peut également utiliser d'autres acides pour former des peroxydes à la surface du matériau prothétique, notamment l'acide fluorhydrique ou l'acide chlorhydrique, toujours en mélange avec $H_2O_2$.

Par « mélange acide/$H_2O_2$ » on entend le mélange simultané ou séquentiel des solutions, à savoir la solution d'acide et la solution de $H_2O_2$. Ainsi, soit les deux solutions sont mises en contact simultanément avec le matériau prothétique à oxyder, soit la solution d'acide est mise en contact dans un premier temps, puis la solution de $H_2O_2$ est mise en contact dans un deuxième temps avec le matériau prothétique. Dans tous les cas, le temps d'oxydation peut varier dans une large mesure et l'homme du métier pourra l'adapter en fonction du matériau prothétique à traiter, du mode d'oxydation chimique mis en oeuvre pour former des espèces donneurs de radicaux libres à la surface de ce matériau et de la densité de greffage souhaitée. L'homme du métier pourra notamment déterminer le meilleur mode opératoire en mesurant le taux de greffage obtenu après avoir greffé des polymères sur le matériau prothétique oxydé à tester, notamment par dosage de la densité de greffage au Bleu de toluidine (voir exemple 1.5).

Dans le cas d'un mélange d'oxydation acide/$H_2O_2$ simultané, de préférence le temps d'oxydation est de préférence de 1 à 10 minutes, de manière plus préférée de 3 à 6 minutes et de manière la plus préférée le temps d'oxydation est de 5 minutes. De préférence, ce temps d'oxydation s'applique à l'oxydation du titane ou d'un de ses alliages avec une solution $H_2SO_4/H_2O_2$.

Dans le cas de l'utilisation d'un mélange séquentiel, le matériau prothétique peut être, par exemple, plongé dans la solution d'acide pendant au moins 10 secondes, de préférence pendant au moins 20 secondes, de préférence pendant au moins 30 secondes, de manière plus préférée pendant plus de 50 secondes, de manière préférée pendant plus de 1 minute, de manière préférée pendant plus de 2 minutes, de manière préférée pendant plus de 3 minutes, de manière préférée pendant plus de 4 minutes. Ce temps de mise en présence du titane, ou d'un de ses alliages, avec $H_2SO_4$ peut être largement supérieur et peut, par exemple, atteindre 30 minutes ou plus. Cependant, dans un mode préféré de réalisation, le temps de mise en présence du matériau prothétique avec la solution d'acide est inférieur ou égal à 5 minutes, temps au delà duquel une diminution du taux de greffage est observée. Cette procédure est en particulier appliquée dans le cas de l'oxydation du titane ou d'un de ses alliages, mis en présence d'une solution de $H_2SO_4$

De la même manière, le temps de mise en présence du matériau prothétique avec la solution de $H_2O_2$ peut varier. De préférence, le matériau prothétique, de préférence le titane ou un de ses alliages, est mis en présence de $H_2O_2$ pendant au moins 10 secondes, de préférence au moins 20 secondes, de préférence au moins 30 secondes, de préférence au moins 40 secondes, de préférence au moins 50 secondes, de préférence au moins 1 minute, de préférence au moins 2 minutes, de préférence pendant 2 à 3 minutes, et de manière la plus préférée pendant deux minutes après ajout de la solution de $H_2O_2$. Dans un mode préféré de réalisation, on préfère une action de $H_2SO_4$ sur le titane, ou un de ses alliages, pendant 1 minute puis une action de $H_2O_2$ sur le titane pendant 2 minutes.

La mise en contact du matériau prothétique avec la solution d'acide et/ou la solution de $H_2O_2$ peut être réalisée par tout moyen connu en soi. Ainsi, par exemple, les solutions peuvent être versées dans un récipient contenant le matériau prothétique, ou le matériau prothétique peut être plongé dans un récipient contenant la/les solution(s).

Sans vouloir être liés par une quelconque théorie, les inventeurs pensent que le traitement par une solution acide permet d'éliminer la couche d'oxyde natif qui se forme spontanément sur les matériaux prothétiques en présence d'air, en l'attaquant chimiquement. Le peroxyde d'hydrogène permet de créer des hydroperoxydes à la surface du matériau prothétique, en particulier des hydroperoxydes de titane (TiOOH) dans le cas de l'utilisation du titane comme matériau prothétique. Ces hydroperoxydes, donneurs de radicaux libres lorsqu'ils sont chauffés, peuvent alors servir d'amorceurs à la polymérisation radicalaire des monomères sur le matériau prothétique, en particulier sur du titane.

La proportion d'acide par rapport à $H_2O_2$ peut varier dans une large mesure et l'homme du métier est à même de définir, sur la base de la présente description, le rapport le plus efficace pour aboutir à un taux de greffage souhaité.

De préférence, une solution $H_2SO_4/H_2O_2$ 50/50 (v/v) est utilisée pour oxyder le matériau prothétique. La température utilisée est généralement la température ambiante (20-30°C), voire une température plus faible (par exemple, entre 0 et 20°C), la réaction d'oxydation pouvant être exothermique.

Avantageusement, on ajoute des sels métalliques (fer, nickel, chrome, cuivre) lors de l'étape de génération des espèces actives donneurs de radicaux libres. Ces sels métalliques sont en particulier ajoutés au mélange acide/$H_2O_2$.

La concentration de ces sels peut varier dans une large mesure. En particulier, la concentration varie de $10^{-4}$ mol/l à 2 mol/l. Ils favorisent la formation en concentration plus importante de peroxyde à la surface du matériau prothétique, en particulier du titane ou d'un de ses alliages, et augmentent ainsi le degré de greffage. Par exemple, on peut avantageusement ajouter au mélange acide/$H_2O_2$ des sels à base de fer, nickel, chrome ou cuivre, comme par exemple le sulfate de fer ($FeSO_4$), l'acétate de fer ($Fe(C_2H_3O_2)_2$, le bromure de fer ($FeBr_3$), l'iodure de fer ($FeI_2$), le nitrate de fer $Fe(NO_3)_3$), le thyocyanate de fer ($Fe(SCN)_2$, $Fe(SCN)_3$) $Fe_2(SCN)_6$, ... De préférence, du sulfate de fer est ajouté au mélange acide/$H_2O_2$.

Avantageusement, une étape d'obtention des radicaux libres à partir des espèces actives générées préalablement à la surface du matériau est réalisée, en particulier par réaction thermique des espèces actives générées. Plus spécifiquement, la réaction thermique est réalisée à une température comprise entre 25 et 160°C, de préférence entre 40 et 100°C, de manière encore plus préférée entre 50 et 80°C, de manière plus préférée entre 60 et 75°C et de manière la plus préférée à 70°C.

L'étape de production des radicaux libres peut être réalisée avant, de préférence pendant ou après l'étape de mise en contact des monomères avec le matériau sur lequel des espèces actives ont été générées.

Ainsi, dans un mode de réalisation particulier, le procédé selon l'invention comprend les étapes suivantes :

- des espèces actives sont générées sur un matériau prothétique tel que défini ci-dessus;
- des radicaux libres sont générés, en particulier par chauffage entre 40°C et 100°C; et
- le matériau prothétique sur lequel des radicaux libres ont été générés est mis en présence d'au moins un monomère porteur d'une fonction permettant une polymérisation radicalaire, sa polymérisation radicalaire permettant la formation d'un polymère bioactif, en l'absence d'oxygène.

Selon un autre mode de réalisation, des espèces actives sont d'abord générées sur le matériau qui est ensuite mis en contact avec des monomères et dans des conditions permettant la formation des radicaux libres à partir des espèces actives, en particulier par chauffage de la solution de monomères.

Dans un autre mode de réalisation particulier de l'invention, des espèces actives sont d'abord générées sur le matériau qui est ensuite mis en contact avec une solution de monomères, les radicaux libres étant alors formés, en particulier par chauffage de la solution de monomères.

Les monomères utilisés dans le présent procédé sont avantageusement greffés sur les supports précités par l'intermédiaire d'une réaction de polymérisation radicalaire. Cette dernière est amorcée par les radicaux libres générés directement sur le support à greffer.

Les monomères utilisés dans le procédé selon l'invention comprennent au moins un groupement qui permet la polymérisation. Avantageusement, les monomères comprennent au moins une insaturation aliphatique, de préférence un groupement vinylique, qui permet la polymérisation. Par monomères présentant au moins une insaturation aliphatique, on entend selon l'invention des monomères présentant une ou deux, de préférence une, double ou triple liaison, avantageusement une double liaison -CH=CH-.

Un avantage du procédé de la présente invention par rapport au procédé indirect décrit ci-dessus repose sur la possibilité de greffer une grande variété de polymères. En effet, tout monomère susceptible d'être polymérisé via une réaction de polymérisation radicalaire peut être utilisé dans la présente invention.

La structure des monomères utilisés dans la présente invention permet la formation d'un polymère bioactif à la surface d'un matériau prothétique. Au sens de l'invention, un polymère est bioactif s'il est capable d'orienter les réponses cellulaires eucaryotes et/ou procaryotes au site d'intégration de l'implant prothétique fabriqué à partir d'un matériau prothétique susceptible d'être obtenu selon le procédé de la présente invention, c'est à dire s'il est capable de favoriser l'ostéointégration de l'implant prothétique et de prévenir le développement d'une infection.

Afin d'améliorer les propriétés d'ostéointégration et anti-bactériennes des matériaux cités ci-dessus, les monomères utilisés dans le cadre du présent procédé comprennent avantageusement un groupement sulfonate et/ou carboxylate. En effet, il a récemment été montré que des polymères porteurs de groupements ioniques sulfonate et/ou carboxylate favorisaient l'adhérence, la colonisation et la différenciation des ostéoblastes. De plus, il a également été montré que des polymères porteurs de ces mêmes groupements, mais plus encore lorsqu'ils sont porteurs des seuls groupements sulfonate, permettaient d'inhiber l'adhérence de souches bactériennes, notamment de *Staphylococcus aureus*, souche très majoritairement impliquée dans les infections sur matériels prothétiques.

On peut également envisager de greffer des groupements osidiques (glucose, saccharose, fructose, polyose,...) ou encore des groupements phosphates de type $-O-PO-(OH)_2$, notamment de formule (A) :

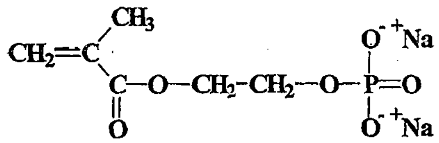

(A)

De préférence, les polymères formés sur les matériaux prothétiques par le procédé selon l'invention sont formés à partir d'au moins un des monomères suivants : l'acide acrylique, l'acide méthacrylique, le méthacrylate de méthyle (MMA), l'acrylamide de N-(sulfonate de sodium phényl) (NaAS), le méthacrylamide de N-(sulfonate de sodium phényl) (NaMS), le styrène sulfonate de sodium (NaSS), l'éthylène glycol méthacrylate phosphate, méthacryloyldi-,isopropylidène, des monomères porteurs de groupements osidiques oses tels que, par exemple, glucose, glucofuranose, saccharose, polyose, fructose, etc.

Les polymères greffés obtenus peuvent être des homopolymères ou des copolymères. Ainsi, dans un mode particulier de réalisation de l'invention, les homopolymères greffés sont formés à partir du styrène sulfonate de sodium (greffage de polyNaSS) ou à partir du méthacrylate de méthyle (greffage de poly (méthacrylate de méthyle), ou PMMA).

Dans un mode particulier de réalisation de l'invention, les polymères susceptibles d'être greffés par le procédé selon l'invention sont des copolymères obtenus par polymérisation radicalaire de monomères comprenant au moins un monomère de formule générale (I) et au moins un monomère de formule générale (II), les formules (I) et (II) étant les suivantes :

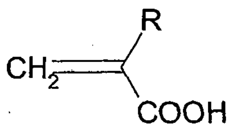

(I)

ou un sel métallique correspondant

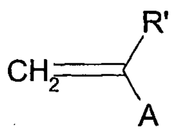

(II),

dans lesquelles R et R', identiques ou différents, représentent un atome d'hydrogène ou un radical alkyle de $C_1$ à $C_6$, linéaire ou ramifié, et A représente un radical de type arylsulfonate ou acide correspondant.

Selon une variante du procédé du procédé selon l'invention, les monomères de formule (I) peuvent au départ présenter des fonctions esters, les copolymères ainsi obtenus seront alors hydrolysés (partiellement ou totalement), pour obtenir les fonctions acide correspondantes.

Plus spécifiquement, A est choisi parmi les radicaux de formules suivantes :

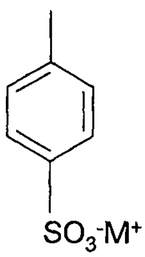

(III) ou

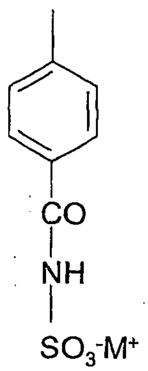

(IV)

dans lesquels M représente un ion métallique, de préférence de métal alcalin.

Le métal alcalin est préférentiellement choisi parmi le sodium et le potassium, avantageusement le sodium.

Parmi les radicaux alkyle de $C_1$ à $C_6$, linéaires ou ramifiés, on peut notamment citer les radicaux méthyle,

éthyle, n-propyle, isopropyle, n-butyle ou t-butyle, pentyle, et hexyle.

Parmi les monomères de formule (I), on peut notamment citer l'acide acrylique (AA), l'acide méthacrylique (MA), l'acide éthacrylique (EA), leurs sels correspondants (en particulier sels de métal alcalin, de préférence de sodium) et leurs mélanges. On préfère l'acide méthacrylique.

Parmi les monomères de formule (II), on peut notamment citer l'acrylamide de N-(sulfonate de sodium phényl) (NaAS), le méthacrylamide de N-(sulfonate de sodium phényl) (NaMS), le styrène sulfonate de sodium (NaSS) et leurs mélanges. On préfère le styrène sulfonate de sodium.

A titre d'illustration, lorsque A du monomère de formule (II) présente la formule (III), le copolymère greffé sur le matériau prothétique peut être représenté plus spécifiquement par la formule générale (V) suivante :

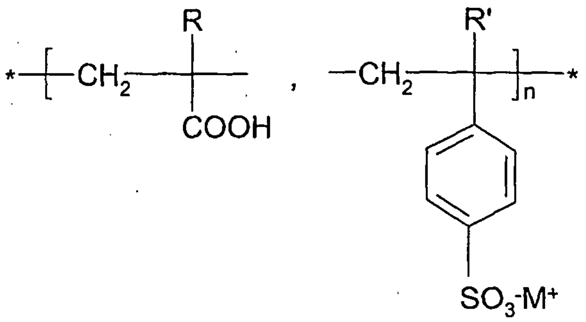

(V)

dans laquelle R, R', et M sont tels que définis ci-dessus, et n représente un nombre réel compris entre 10 et 1000.

La virgule signifie que la quantité, le rapport et l'enchaînement des unités monomères présentes dans les copolymères varient. Cette variation est directement liée aux paramètres du procédé de polymérisation, tels que par exemple la quantité de chaque monomère mis en oeuvre.

Les poids moléculaires des polymères greffés selon le procédé de la présente l'invention peuvent varier dans une large mesure et sont choisis ou contrôlés par l'homme du métier en fonction de leur application ou usage ultérieur. Pour donner un ordre de grandeur, le poids moléculaire moyen en poids peut varier de 1000 à 100 000 daltons.

A ce titre, les quantités des monomères de formules (I) et (II) peuvent varier dans une large mesure et dépend notamment des propriétés désirées pour les copolymères. De préférence, le rapport molaire de (I) sur (II) varie de 90/10 à 10/90, avantageusement de 80/20 à 20/80. Les copolymères selon l'invention présentent en particulier les fractions molaires de (I) sur (II) suivantes : 90/10; 80/20, 70/30, 60/40, 50/50, 40/60, 30/70, 20/80 et 10/90 (ces fractions correspondent en réalité aux fractions des monomères introduits, les fractions des monomères incorporés dans les copolymères obtenus par analyse RMN variant au maximum de $\pm$ 2 par rapport à celles mentionnées ci-dessus).

Les copolymères greffés selon le procédé de la présente invention peuvent être obtenus par polymérisation radicalaire de monomères comprenant, en plus des monomères de formules (I) et (II), d'autres monomères présentant au moins une insaturation aliphatique. Les autres monomères présentant au moins une insaturation aliphatique peuvent être de toute nature, incluant des monomères donnant un caractère hydrosoluble ou non aux polymères greffés. De préférence, les monomères additionnels sont de nature hydrosoluble, tels que les monomères présentant un radical de type sucre ou phosphate.

On peut citer comme monomères additionnels, les monomères de formules suivantes :

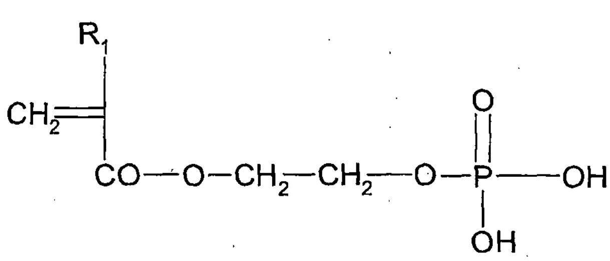

(VI)

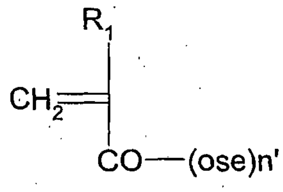

(VII)

dans lesquelles $R_1$, identique ou différent, représente un atome d'hydrogène ou un radical alkyle de $C_1$ à $C_6$, linéaire ou ramifié, tel que défini précédemment, et n' représente un nombre réel compris entre 10 et 1000: Parmi les groupements ose de la formule (VII), on peut notamment citer le glucose, galactose et saccharose.

Afin de préserver les caractéristiques des polymères greffés, l'homme du métier est à même, par des manipulations de routine, de déterminer les monomères additionnels envisageables et dans quelles quantités.

Plus particulièrement et pour donner un ordre de grandeur, la quantité des monomères de formule (I) et (II) est avantageusement supérieure ou égale à 25 %, de préférence supérieure ou égale à 50%, en moles par rapport au nombre total de moles des unités monomères présentes dans les polymères.

Selon un aspect particulier de l'invention, les copolymères sont susceptibles d'être obtenus par polymérisation radicalaire des monomères de formule (I) et (II) uniquement.

Le temps de greffage peut varier dans une large mesure. L'homme du métier est capable de choisir le temps nécessaire à la polymérisation sur le matériau prothétique en fonction de la nature de ce dernier, du polymère à greffer et de la densité de greffage souhaitée. L'homme du métier peut sélectionner le temps le plus adapté par mesure de la densité de greffage au Bleu de Toluidine (voir exemple 1.5).

Avantageusement, le matériau prothétique est mis en contact avec le(s) monomère(s) pendant au moins approximativement 30 minutes, et de préférence entre approximativement 1 heure et approximativement 48 heures. De manière plus préférée, le matériau prothétique est mis en contact du(des) monomère(s) pendant plus de 2 heures, de préférence plus de 3 heures, de préférence plus de 5 heures, de préférence plus de 8 heures, et de préférence pendant approximativement 15 heures.

Dans un mode préféré de réalisation, l'invention concerne un procédé pour greffer du polyNaSS sur du titane comprenant les étapes suivantes :

- des espèces actives donneurs de radicaux libres sont générées à la surface du titane ; et
- le titane sur lequel des espèces actives ont été générées est mis en présence de NaSS à une température comprise entre 40 et 100°C, pendant au moins 8 heures, en l'absence d'oxygène.

Dans un mode particulièrement préféré de réalisation, l'invention concerne un procédé pour greffer du polyNaSS sur du titane comprenant les étapes suivantes :

- des espèces actives donneurs de radicaux libres sont générées à la surface du titane ; et
- le titane sur lequel des espèces actives ont été générées est mis en présence de NaSS à une température de 70°C, pendant 15 heures, en l'absence d'oxygène.

La réaction de polymérisation radicalaire doit se dérouler en l'absence d'oxygène, celui-ci étant un inhibiteur de la réaction. De préférence, la réaction est réalisée en l'absence d'oxygène, sous atmosphère inerte, en particulier sous argon, hélium ou azote, avantageusement sous azote.

Après polymérisation, le matériau ainsi greffé est récupéré, et éventuellement rincé, notamment avec de l'eau (par exemple de l'eau distillée), notamment afin d'éliminer les polymères formés mais non greffés.

Un autre objet de l'invention concerne des matériaux prothétiques greffés avec des polymères bioactifs susceptibles d'être obtenus selon le procédé décrit ci-dessus.

L'invention concerne également l'utilisation d'un matériau prothétique selon l'invention pour la fabrication d'un implant prothétique, en particulier pour la fabrication d'une prothèse articulaire ou d'une prothèse dentaire.

Un objet supplémentaire de l'invention concerne des implants prothétiques fabriqués à partir des matériaux prothétiques susceptibles d'être obtenus par le procédé décrit ci-dessus.

Les avantages de la présente invention vont maintenant être détaillés dans les exemples suivants, et éventuellement à la lumière des figures annexés.

LEGENDE DES FIGURES

**Figure 1 :** diagramme représentant la procédure de greffage de NaSS directe selon l'invention.

**Figure 2 :** graphe représentant le spectre IR de la surface de titane oxydée chimiquement puis greffée de polyNaSS.

**Figure 3A :** déconvolution des éléments carbone C1s (à gauche) et oxygène O1s (à droite) du titane oxydé chimiquement.

**Figure 3B :** déconvolution des éléments carbone C1s (à gauche) et oxygène O1s (à droite) du titane greffé de polyNaSS.

**Figure 3C :** déconvolution des éléments titane Ti2p (à gauche) et soufre S2p (à droite) du titane oxydé chimiquement.

**Figure 3D :** déconvolution des éléments titane Ti2p (à gauche) et soufre S2p (à droite) du titane greffé de polyNaSS.

**Figure 3E :** comparaison des déconvolutions de l'élément soufre S2p du titane oxydé chimiquement (à gauche) et du titane greffé de polyNaSS (à droite).

**Figure 4 :** vues en coupe de la surface osseuse néoformée autour d'implants. *S100/0C : 100% sulfonate et 0% carboxylase ; C100/S0 100% carboxylase et 0% sulfonate.*

## EXEMPLES

### Exemple 1 : Greffage de polymère bioactif à la surface de cylindres en titane

La première étape a été la mise au point du greffage des groupements ioniques. Par souci de simplification, nous avons choisi dans un premier temps de greffer uniquement des groupements arysulfonate. La fonctionnalisation a été faite sur des disques de titane (99,7% de pureté) préalablement polis pour s'affranchir des problèmes de rugosité.

Le greffage des groupements arysulfonate à la surface du titane s'est fait par voie directe par création de radicaux libres par oxydation chimique qui sont ensuite utilisés comme amorceurs de la polymérisation d'un monomère porteur des fonctions arylsulfonate.

La molécule de monomère est le styrène sulfonate de sodium (NaSS). L'ensemble de la procédure adoptée est résumé dans le figure 1.

#### 1.1. Caractéristiques du titane

Le titane *(société Alfa Aesar)* se présente sous la forme de barreaux extrudés de 12,7 mm de diamètre, ayant subi un recuit de détente de 30 min à 700°C. Sa pureté (Tableau 1) est de 99,7% de composition nominale (grade 1, ou T40).

Tableau 1 Composition chimique du titane pur utilisé

| Elément | C | N | O | H | Fe | Ti |
|---|---|---|---|---|---|---|
| % atomique | 0,006 | 0,005 | 0,14 | 0,0014 | 0,08 | 99,7676 |

Toutes les études ont été réalisées sur des disques de 2 mm d'épaisseur, obtenus par découpe par électroérosion dans un bain d'eau réalisé par la société Prestasem (Champagne sur Seine, France 77). Ce mode de découpe présente l'avantage d'éviter un écrouissage trop important du matériau et une perte de matière trop grande, par rapport à un usinage au tour par exemple.

#### 1.2. Polissage du titane

Le polissage mécanique des disques est réalisé à l'aide d'un bras automatique, monté sur une polisseuse rotative, avec du papier abrasif de granulométries décroissantes *(Struers).* Un premier polissage avec un papier de grade 800 (grains de 22 μm) enlève environ 1/10$^{e}$ de mm d'épaisseur, ce qui a pour effet de retirer l'épaisseur de métal abîmée par les piqûres dues au mode de découpe par électroérosion (arc électrique).

Le polissage est ensuite affiné par l'utilisation de papiers de granulométries de plus en plus faibles (papiers 1000, puis 1200). Le protocole utilisé est le suivant : 8 min à P800, 4 min à P1000, 4 min à P1200 (grains de 14 μm).

On obtient une surface de rugosité équivalente à celle qui est couramment utilisée dans la littérature pour les études de comportement cellulaire.

Après polissage, les échantillons sont lavés avec une solution d'acétone pure sous ultrasons pendant 10 minutes à température ambiante. Ils sont ensuite séchés et utilisés immédiatement, ou conservés sous argon (boîte à gants).

**1.3. Purification du monomère NaSS**

Le monomère utilisé pour apporter les groupements ioniques adéquats à la surface du titane est le styrène sulfonate de sodium (NaSS, sel sodique d'acide 4-vinylbenzenesulfonique, Aldrich), monomère vinylique dont la formule chimique est représentée ci-dessous

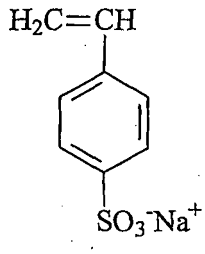

**Molécule de Styrène Sulfonate de Sodium**

Le styrène sulfonate de sodium est purifié par recristallisation. Cette procédure consiste à dissoudre une masse m de monomère dans un mélange eau/éthanol en proportion 10/90 à la température de 60-70°C. La solution est ensuite filtrée à chaud, sous vide de trompe à eau. La solution ainsi filtrée est placée à 4°C pendant 24h. Les cristaux formés sont récupérés par filtration sous vide, puis séchés à 60-70°C sous vide.

**1.4. Oxydation et greffage**

La fonctionnalisation se fait sous atmosphère contrôlée d'argon (boîte à gants) et peut se résumer comme suit :

1. Nettoyage des surfaces de titane polies ;
2. Formation de radicaux peroxydes à la surface des disques en titane par trempe des échantillons dans un mélange $H_2SO_4$ (*Acros*)/$H_2O_2$ (*Aldrich*) 50/50 à température ambiante. Les deux réactifs sont versés simultanément dans le bécher contenant les échantillons, et l'ensemble est agité pendant 2 minutes. La réaction est immédiate, fortement exothermique et s'accompagne d'un dégagement gazeux. La solution d'oxydation initialement incolore vire au jaune, à l'orangé, puis au rouge brique (ce qui est dû à la réaction de l'acide sulfurique sur le titane et la formation d'ions $Ti^{4+}$).
3. Rinçages multiples des surfaces oxydées à l'eau distillée ;
4. Immersion des échantillons dans une solution aqueuse de monomère NaSS à la concentration de 0,7 M ;
5. Chauffage à 70°C de la solution de monomères en contact avec les échantillons de titane pendant 14 h. Le chauffage à 70°C permet la rupture de la liaison peroxyde (O-O) en radicaux libres *TiO*•, qui serviront à amorcer la polymérisation radicalaire des monomères vinyliques ;
6. Rinçage des surfaces métalliques greffées de polyNaSS à l'eau distillée afin d'éliminer l'homopolymère formé en solution, susceptible d'être mêlé avec les chaînes de polymère greffées.

**1.5. Caractérisation chimique et physico-chimique**

**a) Dosage de la densité de greffage au Bleu de Toluidine**

Le « dosage au bleu de Toluidine » est une technique colorimétrique utilisant la molécule chromophore est le Bleu de Toluidine qui absorbe dans le visible à 633 nm. Cette molécule a la particularité de se complexer, via son groupement $N^+(CH3)_2$, avec les groupements anioniques ($-COO^-$). Une mole de Bleu de Toluidine se complexe avec une mole de groupement carboxylate. Le dosage du bleu de toluidine donne donc la quantité de monomère greffé. Cette propriété a été transposée au NaSS, qui peut se complexer au groupe $N^+(CH3)_2$ via son groupement sulfonate ($-SO_3^-$).

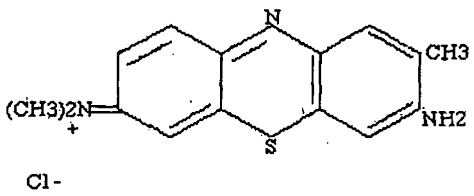

Les échantillons de titane greffés sont mis au contact d'une solution de Bleu de Toluidine à $5.10^{-4}$ M (ajustée à pH 10) à la température de 30°C pendant 6 heures. Cette étape correspond à la complexation du Bleu de Toluidine aux unités monomères du polyNaSS greffées. Les échantillons sont ensuite rincés abondamment avec une solution d'hydroxyde de sodium $1.\ 10^{-4}$ M, pour éliminer le Bleu de Toluidine non complexé. On arrête les rinçages lorsque la solution est incolore.

Le Bleu de Toluidine complexé est ensuite décomplexé au moyen d'une solution d'acide acétique 50% (*Acros organics*) qui est laissée au contact des échantillons de titane pendant 24h.

La solution obtenue est dosée par spectrophotométrie à l'aide d'un spectrophotomètre de type Safas Xenius piloté par son logiciel. Le coefficient d'extinction molaire $\varepsilon$ du bleu de toluidine est égal à 51230 $L.mol^{-1}.cm^{-1}$.

A partir de la concentration calculée on calcule la quantité de molécules greffées à partir de l'équation suivante :

$$m = [Monomère] \times V \times M_{monomère} \text{ (en g)}$$

avec V = volume de la solution dosée ($5.10^{-3}$ L)
$M_{monomère}$ = masse molaire du monomère NaSS

La surface d'une pastille mesure 1 $cm^2$, donc cette masse de monomère greffé peut être exprimée en quantité de matière greffée en $g/cm^2$ pour une face de pastille. La densité optique mesurée donne une quantité de molécules greffées de 10 $\mu g/cm^2$ environ.

Les échantillons dosés ont ensuite été relavés plusieurs fois à l'eau et leur complexation au bleu de toluidine a été dosée jusqu'à quatre fois. La densité de greffage mesurée est alors tombée à 3 $\mu g/cm^2$, et s'est stabilisée à cette valeur. Le phénomène exposé précédemment peut expliquer cette observation : en effet, la complexation au Bleu de Toluidine a peut-être entraîné une distorsion des chaînes de polymère et les multiples lavages effectués ont permis d'évacuer les chaînes enchevêtrées et non réellement greffées.

La densité de greffage obtenue avec le procédé selon l'invention est une densité extrêmement importante car les valeurs obtenues dans la littérature avec le procédé indirect par silanisation sont plutôt de l'ordre de 0,1 à 0,2 $\mu g/cm^2$.

**b) <u>Spectroscopie InfraRouge à Transformée de Fourier à Réflexion Totale Atténuée (ATR/FTIR)</u>**

Nous avons utilisé la spectroscopie FTIR en mode de réflexion totale atténuée (ATR) pour analyser la surface de nos échantillons en titane. Le spectrophotomètre FTIR utilisé pour réaliser les mesures est un appareil Nicolet, Avatar 370-FT-IR. Les échantillons sont analysés en mode ATR. Pour les mesures, les longueurs d'ondes sont balayées entre 4000 et 650 $cm^{-1}$, au pas de 4 $cm^{-1}$, avec une résolution de 1 $cm^{-1}$. Les échantillons de titane, oxydés ou non, greffés ou non, sont appliqués avec une force calibrée contre le cristal, afin d'assurer un contact maximal entre l'échantillon et le diamant ; 120 acquisitions sont accumulées par point, pour limiter le bruit de fond au maximum. Le background est pris sur l'atmosphère.

Le tableau 2 ci-dessous résume les principales bandes d'absorption caractéristiques du polyNaSS et leurs attributions aux différents groupements chimiques.

Tableau 2 : Bandes d'absorption IR caractéristiques du polyNaSS

| Fréquence ($cm^{-1}$) | Apparence du (ou des) pic(s) | Attribution |
|---|---|---|
| 1640-1600 et 1496-1433 | Faible | $\nu$ (C=C) du noyau aromatique |
| 1410 | Moyen | $\nu(SO_2)$ |
| 1180-1127 | Fort | $SO_3^-Na^+$ (sel) |
| 1040 | Fort | $\nu$(O=S=O) |
| 1009 | Fort | noyau aromatique |

Les pics caractéristiques des vibrations du groupement sulfone O=S=O et du noyau aromatique de la molécule de NaSS constituent un doublet situé à 1009-1040 $cm^{-1}$; les pics situés vers 1127 et 1180 $cm^{-1}$ sont attribuables à l'absorption du sel d'acide sulfonique. La vibration de valence antisymétrique du groupement $SO_2$ correspond au pic à 1410 $cm^{-1}$, et on note un épaulement du pic précédent vers 1220 $cm^{-1}$, ce qui correspond à la vibration de valence symétrique du groupement $SO_2$. Quant à la série de pics entre 1640 et 1430 $cm^{-1}$, elle peut être attribuée aux vibrations de valence des liaisons (>C=C<) du noyau benzénique.

Le spectre infrarouge de la surface de titane oxydée chimiquement et greffée de ce même produit est présenté sur la figure 2.

La présence de polyNaSS sur cette surface est surtout confirmée par les deux pics caractéristiques à 1008 et 1040 $cm^{-1}$. On retrouve aussi l'absorption du sel d'acide sulfonique vers 1127-1180 $cm^{-1}$. En revanche, les vibrations de valence du groupement $SO_2$ et des doubles liaisons C=C du noyau aromatique sont noyées dans le bruit de fond, compte tenu de la différence d'intensités entre le produit pur de polyNaSS sous forme de poudre et le produit greffé sur la surface.

c) <u>Analyse chimique par spectroscopie des photoélectrons (XPS)</u>

Les échantillons ont été analysés avec un spectromètre Escalab VG 220i-XL. Les rayons X sont produits par une source monochromatique AlK$\alpha$ de 1486,6 eV.

Les échantillons sont introduits quatre par quatre dans la chambre d'analyse, qui est pompée sous ultra vide ($10^{-9}$ à $10^{-10}$ mbar). L'angle d'émission utilisé est de 45°, pour une profondeur d'analyse de la surface de 10 nm en pleine profondeur (le détecteur étant placé à la normale de l'échantillon). Le diamètre du pointé sur l'échantillon est de 150 à 200 $\mu$m (puissance de 70 W environ, faisceau incident 10 kV).

L'analyse de chaque échantillon commence par l'acquisition d'un spectre global balayant tout le domaine des énergies de liaison, afin d'identifier les principaux éléments chimiques présents à la surface de l'échantillon, et qui seront analysés chacun ensuite plus finement. La résolution en énergie donnée est de $\pm$ 0,1 eV.

En ce qui concerne l'analyse des échantillons simplement oxydés, les éléments principaux sont le carbone, l'oxygène, et le titane ; pour les échantillons greffés, le soufre et le sodium (NaSS) sont aussi détaillés. Les déconvolutions sont réalisées avec le logiciel Elipse, fourni par VG ; la référence est prise sur le pic principal de la contribution $C_{1s}$ du carbone, que l'on fixe à une énergie de liaison de 284,8 eV.

La composition chimique des surfaces de titane oxydé chimiquement et greffées a été reportée dans le tableau 3.

Tableau 3 : composition chimique des surfaces de titane

| % atomiques $\pm$ SD | C | O | Ti | S | Na | Polluants |
|---|---|---|---|---|---|---|
| **Témoin Ti poli P1200** | 33,2 $\pm$ 4,4 | 46,4 $\pm$ 2,1 | 14,7 $\pm$ 0,4 | - | - | 5,4 $\pm$ 2,2 |
| **Oxyde chimique $H_2SO_4/H_2O_2$** | 34,2 $\pm$ 2,6 | 47,0 $\pm$ 1,6 | 7,4 $\pm$ 0,8 | 7,8 $\pm$ 1,7 | - | 2,7 $\pm$ 0,6 |
| **Oxyde chimique greffé de pNaSS** | 53,8 $\pm$ 1,3 | 30,3 $\pm$ 1,9 | 5,0 $\pm$ 1,4 | 5,8 $\pm$ 0,9 | 3,8 $\pm$ 0,8 | 1,2 $\pm$ 0,4 |

La composition globale de surface de l'oxyde chimique est quasiment la même que celle de l'oxyde natif du témoin, à l'exception du pourcentage atomique du titane qui est plus faible : ainsi, les pourcentages de carbone et d'oxygène sont respectivement de 33% et 46% pour l'oxyde natif et de 34% et 47% pour l'oxyde chimique. Par contre, le pourcentage atomique de titane chute de 15% à 7% après oxydation chimique. En fait, la quantité de titane détectée diminue en « proportion relative » à cause de la présence importante de contamination au soufre. Ce soufre détecté en grande quantité (8% atomiques) sur les échantillons oxydés, est probablement un résidu du bain d'oxydation qui contient de l'acide sulfurique qui persiste malgré tous les rinçages effectués.

L'analyse de l'évolution de composition atomique entre l'état oxydé et l'état greffé de polyNaSS permet de faire les constats suivants :

- Le pourcentage atomique de carbone augmente d'un facteur 1,6, et passe de 34% pour l'oxyde chimique à 54% pour l'oxyde greffé.
- Parallèlement, le pourcentage d'oxygène diminue d'un facteur 1,6, et sa contribution passe de 47% à 30%. Par conséquent, le rapport C/O passe de 0,7 pour l'oxyde seul à 1,8 pour l'oxyde greffé de polyNaSS. Cela semble logique, étant donné que les molécules de NaSS ajoutées contiennent peu d'oxygène par rapport au carbone.
- La contribution du titane diminue elle aussi de 7,4% à 5%, car l'oxyde est masqué et le titane plus difficilement détecté sous l'épaisseur de polymère greffé.
- Apparition de sodium (4%), et maintien du pourcentage de soufre à 6%, par rapport à l'oxyde seul (8%).

Ces résultats confirment qu'il y a eu polymérisation du NaSS et que du polyNaSS est bien présent en surface du titane.

Par ailleurs, le rapport des pourcentages atomiques Na/S oscille entre 0,6 et 0,7, alors qu'en toute logique il devrait être de 1. Cela peut être expliqué par le fait que le styrène sulfonate de sodium NaSS est un sel qui est dissocié en milieu aqueux, et il est donc possible que le sodium soit complexé à un autre contre ion.

Les déconvolutions des différentes contributions des éléments C1s, O1s, Ti2p et S2p des échantillons oxydés chimiquement et greffés de polyNaSS sont données dans les figures 3A-3E.

L'allure de la déconvolution du spectre C1s du carbone (à gauche) de l'oxyde greffé (figure 3B) ressemble sensiblement à celle de l'oxyde non greffé (figure 3A). Cependant, le pourcentage très important d'espèces hydrocarbonées à 284,6 eV est attribué à la chaîne carbonée et aux cycles du polyNaSS greffé (liaisons CHx et C=C). Cette contribution représente à présent la quasi-totalité (85%) du carbone présent.

Le reste est partagé entre les composantes liées à l'oxygène, dans des intensités beaucoup plus faibles que précédemment (15% du carbone présent contre 35% pour l'oxyde seul).

Sur le spectre O1s de l'oxygène (à droite), la contribution habituellement hydroxyle à 532 eV est décalée vers les basses énergies à 531,5 eV. Elle peut alors être attribuée aux ions $SO_3^-$ des groupements fonctionnels sur les cycles du polyNaSS; cette composante (50% de l'oxygène présent) regroupe aussi les carbonyles C=O et potentiellement une partie des hydroxyles OH s'ils sont encore présents.

La déconvolution du spectre de Ti2p (à gauche) de l'oxyde greffé (figure 3D) a la même allure qu'avec l'oxyde non greffé (figure 3C), avec une composante Ti(IV) unique et encore moins intense, car le titane présent dans l'oxyde $TiO_2$ s'est éloigné de la zone analysé, masqué par la couche de polymère.

La position du pic du soufre à 168,1 eV (avec la correspondance sur O1s à 531,5 eV) montre que le soufre est présent sous forme d'ions sulfonate $SO_3^-$. Le pic $S2p_{1/2}$ est positionné avec un $\Delta$ en énergie de 1,18 eV, soit vers 169,3 eV.

La figure 3E reporte pour comparaison les spectres $S2p_{3/2}$ du soufre, obtenus respectivement sur l'oxyde chimique seul (à gauche) et l'oxyde chimique greffé (à droite). On observe aisément le décalage de la position du pic $S2p_{3/2}$ de 168,9 eV à 168,1 eV entre les états oxydé et greffé. Cela atteste que le soufre de l'échantillon greffé de polyNaSS est présent sous forme d'ions sulfonate $SO_3^-$, et non plus sulfate $SO_4^{2-}$, ce qui confirme à nouveau le greffage du polyNaSS.

Le pic du Na1s du sodium sort à une énergie de 1072 eV.

**d) <u>Energie de surface par mesure de l'angle de contact</u>**

Une goutte de liquide est posée sur une surface, et après obtention de la position d'équilibre, l'angle entre la tangente de la goutte et la surface est déterminée à l'aide d'un goniomètre.

Les mesures d'angle de contact ont été réalisées par la « méthode de la goutte posée », en mode statique, à l'aide d'un appareil DSA 10 (*Kruss*). L'énergie de surface a été déduite des mesures de ces angles par la méthode d'Owens-Wendt.

$$\frac{\gamma_L(\cos\theta + 1)}{2\sqrt{\gamma_L^d}} = \sqrt{\gamma_S^p} * \sqrt{\frac{\gamma_L^p}{\gamma_L^d}} + \sqrt{\gamma_S^d}$$

$$\frac{\gamma_L(\cos\theta + 1)}{2\sqrt{\gamma_L^d}} = \sqrt{\gamma_S^p} * \sqrt{\frac{\gamma_L^p}{\gamma_L^d}} + \sqrt{\gamma_S^d}$$

Des gouttes de 0,5 µL de différents solvants sont déposées sur les surfaces : eau distillée, formamide (à 99,5%, *Aldrich*), éthylène glycol et diiodométhane. Les mesures sont prises une fois l'état d'équilibre atteint après le dépôt de chaque goutte, soit environ 5 secondes. Au moins six zones de la surface sur deux échantillons différents élaborés selon le même protocole sont ainsi photographiées pour vérifier l'homogénéité des surfaces étudiées. L'erreur expérimentale est estimée à ± 2°. Les résultats des mesures sont représentés dans le tableau 4.

Tableau 4 : Angles de contact mesurés sur les surfaces de titane oxydées chimiquement et greffées de polyNaSS par voie directe

| Angles de contact | Eau | Formamide | Ethylène Glycol | Diiodométhane |
|---|---|---|---|---|
| Témoin titane | 59 ± 5 | 35 ± 5 | 38 ± 5 | 48 ± 5 |
| Oxyde chimique | 31 ± 7 | 18 ± 4 | 20 ± 6 | 30 ± 6 |
| Oxyde chimique + pNaSS | 16 ± 5 | 18 ± 5 | 17 ± 4 | 40 ± 5 |

On constate une diminution de la valeur de l'angle de contact avec l'eau en passant du titane témoin au titane greffé de poly NaSS due au caractère hydrophile du poly NaSS.

A partir des valeurs d'angles de contact, les énergies de surface correspondantes ont été calculées par la méthode d'Owens-Wendt (Tableau 5).

Tableau 5

| Energie de surface | Energie de surface globale $\gamma$ (mN/m) | Composante dispersive $\gamma^d$ (mN/m) | Composante polaire $\gamma^p$ (mN/m) |
|---|---|---|---|
| Témoin titane | 44,3 (±2,3) | 27,7 (±1,1) | 16,5 (±1,2) |
| Oxyde chimique | 59,2 (±2,3) | 26,1 (±1,0) | 33,1 (±1,3) |
| Oxyde chimique + pNaSS | 64,9 (±1,9) | 16,9 (±0,7) | 47,9 (±1,2) |

On constate que la valeur globale de l'énergie de surface augmente de plus en plus en passant du titane poli au titane greffé de polyNaSS. Ainsi, elle passe de 44,3 mN/m pour le titane non modifié à 59,2 mN/m pour le titane oxydé, et à 64,9 mN/m pour le titane greffé. Cette augmentation est principalement due à l'augmentation de la composante polaire de l'énergie dont la contribution est multipliée par 2 entre les états non modifié et oxydé ; cette même contribution augmente pratiquement d'un facteur 3 entre les états non modifié et greffé, tandis que celle de la composante dispersive diminue dans le même temps d'un facteur 1,6.

L'oxydation chimique au peroxyde d'hydrogène provoque en effet l'apparition de groupements TiOH augmentant ainsi le caractère hydrophile de la surface du titane.

Ensuite, le greffage du polyNaSS augmente légèrement l'énergie de surface par comparaison avec le titane oxydé chimiquement. L'énergie de surface passe de 59,2 à 64,9 mN/m, le NaSS étant une molécule polaire grâce à son groupement sulfonate ionique, et porteur de trois atomes d'oxygène.

**Expérience annexe :**

Le greffage de polyméthacrylate de méthyle (PMMA) a été réalisé de manière identique à celui du poly NaSS. La mesure de l'angle de contact d'une goutte d'eau sur du titane greffé avec du PMMA a donné une valeur de 65° proche de la valeur de 70° obtenu sur du PMMA pur. Cette expérience confirme qu'il est possible de greffer différents polymères à la surface du titane.

**Exemple 2 : Amélioration de la densité de greffage de polyNaSS**

Afin d'optimiser la quantité de polymère greffé sur le titane, différents paramètres ont été modifiés dans le procédé de l'exemple 1 ci-dessus. Ainsi, une densité aussi élevée que 15,4 $\mu g/cm^2$, telle que mesurée par dosage au Bleu de Toluidine, a été obtenue avec les paramètres suivants (procédé A) :

- oxydation par utilisation d'un mélange séquentiel : $H_2SO_4$ pendant 1 minute puis $H_2O_2$ pendant 2 minutes ;
- utilisation de sulfate de fer comme catalyseur dans le mélange $H_2SO_4$/ $H_2O_2$ ; et
- réaction de polymérisation à 70°C pendant 15 heures.

Avec un tel procédé, la densité de greffage obtenue est particulièrement élevée par rapport aux densités susceptibles d'être obtenues avec le procédé indirect par silanisation de l'art antérieur. De plus, le greffage obtenu avec le procédé A est stable puisque après 1 mois, aucune perte de polymère à la surface du titane n'a été observée (Tableau 6)

Tableau 6 : stabilité du greffage par le procédé A

| | Densité de greffage (μg/cm²) 1 jour | Densité de greffage (μg/cm²) 2 jours | Densité de greffage (μg/cm²) 1 mois |
|---|---|---|---|
| Procédé A | 15.4±3.8 | 15.5±3.5 | 15.5±3.4 |

**Exemple 3 : Activité cellulaire au contact des surfaces modifiées**

***A) Développement d'ostéoblastes à la surface de titane modifiées***

Les cellules choisies pour étudier l'activité cellulaire au contact des surfaces de titane modifiées appartiennent à une lignée dérivée d'ostéoblastes humains MG-63. Nous avons réalisé des mesures d'adhérence, d'activité phosphatase alcaline et de quantités de phosphate de calcium précipité.

Les mesures d'adhérence des cellules MG63 ont été réalisées après 30 minutes d'incubation sous une force constante de 140 dynes/cm$^2$. On constate que le pourcentage de cellules détachées diminue du titane oxydé (14%) au titane greffé de poly NaSS (8%). Les surfaces greffées de polyNaSS améliorent l'adhérence bien que leur caractère hydrophile soit d'un niveau assez proche de celui des surfaces seulement oxydées.

Des mesures d'adhérence réalisées 4 heures après l'incubation montre des résultats similaires bien que les différences soient moins marquées.

La différenciation cellulaire a également été étudiée à travers deux marqueurs principaux : l'activité phosphatase alcaline et la minéralisation.

La première étape a été de mesurer l'activité de la phosphatase alcaline des cellules pour le rôle primordial de cette enzyme dans la formation de l'os et son effet inducteur sur le dépôt minéral car elle régule le transport de phosphate. La présence de poly NaSS greffé à la surface du titane augmente de manière significative l'activité phosphatase alcaline de l'ordre de 25% par rapport à la surface de titane oxydé.

La deuxième étape de la différenciation se caractérise par la formation d'os et de dépôt minéral: la calcification. Le dosage de phosphate de calcium a donc été réalisé pour déterminer le stade de différenciation atteint par les ostéoblastes MG-63. Il a été constaté que les quantités de calcium extraites des cellules sont augmentées de manière significative (environ 30%) lorsque les supports ont été greffés de polyNaSS par rapport aux surfaces oxydées.

Les derniers résultats obtenus concernent l'implantation de prothèse en titane modifié avec soit du polyNaSS, soit du poly acide méthacrylique ou le mélange des deux polymères. Ces essais préliminaires ont été faits avec des échantillons de titane qui n'ont pas été caractérisés dé façon aussi pointue que les échantillons greffés de polyNaSS lors de l'étude de l'adhérence d'ostéoblastes humains MG-63. Le but de l'étude a été d'évaluer in vivo la réponse tissulaire du titane greffés avec des groupements sulfonate ou carboxylate. Pour atteindre cet objectif, des cylindres en titane modifié ou non modifié ont été implantés au niveau du condyle fémoral de lapins qui ont été sacrifiés au bout de 4 semaines. Une quantification de la surface osseuse néoformée autour des implants est évaluée à partir d'une coupe de chaque échantillon grâce à un analyseur d'image (figure 4).

D'une manière semi quantitative, on peut noter que sur l'échantillon recouvert de poly NaSS, l'os est réparti régulièrement le long de la surface osseuse avec des surfaces de contact relativement étendues. On n'observe pas de tissu fibreux et de nodules cartilagineux contrairement aux implants en titane non modifié.

Pour la composition qui contient uniquement des groupements carboxylate, les contacts entre l'os et l'implant sont présents mais les surfaces sont plus petites et moins nombreuses. Il existe des zones de tissu fibreux au contact de la surface implantée qui contiennent des cellules géantes.

La présence de groupement sulfonate augmente la quantité de tissu osseux en contact avec l'implant. Inversement, la présence de groupements carboxylate favorise la formation de tissu fibreux.

Une évaluation plus quantitative du contact os/implant a été réalisée par histomorphométrie. Le pourcentage moyen de contact os/implant pour le titane non modifié était de 32% et de 38% pour le titane modifié avec du sulfonate. Le pourcentage de contact diminue fortement pour l'implant recouvert de groupement carboxylate (12%). Le pourcentage de contact cartilage/implant varie de 1.3% pour le titane $SO_3^-$ à 8% pour titane carboxylé. Le point important est que le pourcentage de contact os + cartilage pour le titane sulfonaté est supérieur à celui mesuré pour le titane non modifié.

***B)- Inhibition de l'adhérence bactérienne***

Au sujet de l'adhérence bactérienne quelques mesures avec *Staphylococcus aureus* ont été réalisées. Nous avons mesuré des taux d'inhibition d'adhérence bactérienne de 79% par rapport au titane poli et de 54% par rapport

au titane oxydé. Ces premiers résultats très encourageants ont confirmé ceux obtenus lors de mesure d'adhérence de *Staphylococcus aureus* et de *Streptococcus pyogenes* sur du polyNaSS. Ils ont été confirmés récemment sur des implants en silicone recouverts de groupements sulfonate qui permettait de diminuer in vivo l'adhérence bactérienne de 2 unités de log.

**Revendications**

1. Procédé de greffage de polymères bioactifs sur un matériau prothétique en titane ou en alliage de titane comprenant les étapes suivantes :

   - la génération d'espèces actives donneuses de radicaux libres à la surface du matériau prothétique en titane ou en alliage de titane par oxydation dudit matériau prothétique en titane ou en alliage de titane ;
   - la génération de radicaux libres à la surface du matériau prothétique en titane ou en alliage de titane par réaction thermique à une température comprise entre 40 et 100°C; et
   - la mise en présence dudit matériau prothétique en titane ou en alliage de titane sur lequel des radicaux libres ont été générés avec au moins un monomère porteur d'une fonction permettant une polymérisation radicalaire, la polymérisation radicalaire dudit monomère permettant la formation d'un polymère bioactif, ladite polymérisation radicalaire étant réalisée en l'absence d'oxygène.

2. Procédé selon la revendication 1, dans lequel les espèces actives donneurs de radicaux libres sont générées à la surface du matériau prothétique par oxydation avec un mélange acide/$H_2O_2$.

3. Procédé selon la revendication 2, dans lequel le matériau prothétique est un alliage de titane, en particulier un alliage à base de nickel, de vanadium, d'aluminium, de zirconium et/ou de molybdène.

4. Procédé selon l'une quelconque des revendications précédentes, dans lequel les radicaux libres sont obtenus par réaction thermique à une température de 70°C.

5. Procédé selon l'une quelconque des revendications précédentes, dans lequel les radicaux libres sont obtenus avant, de préférence pendant ou après l'étape de mise en contact des monomères avec ledit matériau.

6. Procédé selon l'une quelconque des revendications précédentes, dans lequel le monomère présente au moins une double ou triple liaison, en particulier dans lequel le monomère porte un groupement vinylique.

7. Procédé selon l'une quelconque des revendications précédentes, dans lequel, au moins un monomère porte une fonction carboxyle ou sulfonate.

8. Procédé selon la revendication 6, dans lequel le monomère est l'acide acrylique, l'acide méthacrylate, le méthacrylate de méthyle (MMA), l'acrylamide de N-(sulfonate de sodium phényl) (NaAS), le méthacrylamide de N-(sulfonate de sodium phényl) (NaMS), le styrène sulfonate de sodium (NaSS), l'ethylène glycol methacrylateméthacrylate phosphate, le methacryloyl-di-isopropylidene, les monomères porteurs de groupements osidiques tels que le glucose, le glucofuranose, le saccharose, le polyose, le fructose ou tout mélange de ces composés.

9. Procédé selon la revendication 8, le monomère étant le styrène sulfonate de sodium (NaSS).

10. Procédé selon l'une quelconque des revendications précédentes, dans lequel ladite réaction radicalaire est réalisée en l'absence d'oxygène, pendant au moins approximativement 30 minutes, de préférence pendant entre 1 et 48 heures, de préférence pendant approximativement 15 heures.

11. Procédé selon l'une quelconque des revendications précédentes, pour le greffage de polyNaSS sur du titane, comprenant:

    - l'oxydation du titane pour générer à la surface dudit titane des espèces actives donneuses de radicaux libres;
    - la mise en présence du titane sur lequel des espèces actives ont été générées avec du NaSS à une température comprise entre 40 et 100°C; pendant au moins 8 heures, en l'absence d'oxygène.

12. Procédé selon la revendication 11, comprenant:

- l'oxydation du titane pour générer à la surface dudit titane des espèces actives donneuses de radicaux libres;
- la mise en présence du titane sur lequel des espèces actives ont été générées avec du NaSS à une température de 70°C, pendant 15 heures, en l'absence d'oxygène.

**13.** Procédé selon l'une quelconque des revendications précédentes, dans lequel le matériau prothétique est poli avant formation des espèces actives à sa surface, en particulier avec du papier abrasif, de préférence avec différents papiers abrasifs de granulométrie décroissante jusqu'à P1200, notamment des papiers abrasifs successifs de granulométrie P800, P1000 et P1200.

**14.** Procédé selon l'une quelconque des revendications précédentes, dans lequel le mélange acide/$H_2O_2$ est un mélange séquentiel comprenant la mise en contact du matériau prothétique avec une solution d'acide puis la mise en contact avec $H_2O_2$.

**15.** Procédé selon l'une quelconque des revendications précédentes, dans lequel l'acide est l'acide fluorhydrique, l'acide chlorhydrique ou $H_2SO_4$, de préférence $H_2SO_4$.

**16.** Procédé selon la revendication 15, dans lequel le matériau prothétique est le titane ou un de ses alliages qui est mis en contact avec une solution acide de $H_2SO_4$ pendant 1 minute puis en contact d'une solution de $H_2O_2$ pendant 2 minutes, le rapport $H_2SO_4/H_2O_2$ étant de préférence de 50/50 (v/v).

**17.** Procédé selon l'une quelconque des revendications 2 à 16, dans lequel des sels métalliques, en particulier des sels métalliques choisis parmi les sels de fer, de nickel, de chrome et de cuivre, sont ajoutés au mélange acide/$H_2O_2$, les sels métalliques étant plus particulièrement ajoutés à une concentration allant de $10^{-4}$ mol/l à 2 mol/l.

**18.** Procédé selon la revendication 17, dans lequel le sel métallique est le sulfate de fer.

**19.** Matériau prothétique en titane ou en alliage de titane sur lequel est greffé un polymère bioactif susceptible d'être obtenu suivant le procédé de l'une quelconque des revendications 1 à 18.

**20.** Utilisation d'un matériau prothétique en titane ou en alliage de titane selon la revendication 19, pour la fabrication d'un implant prothétique, en particulier pour la fabrication d'une prothèse articulaire ou d'une prothèse dentaire.

**21.** Implant prothétique **caractérisé en ce qu'**il est fabriqué à partir d'un matériau prothétique en titane ou en alliage de titane selon la revendication 19.

## Patentansprüche

**1.** Verfahren zur Implantation bioaktiver Polymere in einem Prothesenmaterial aus Titan oder einer Titanlegierung, umfassend die folgenden Schritte:

- die Erzeugung aktiver Donorspezies freier Radikale an der Oberfläche des Prothesenmaterials aus Titan oder einer Titanlegierung durch Oxidation besagten Prothesenmaterials aus Titan oder einer Titanlegierung;
- die Erzeugung freier Radikale an der Oberfläche des Prothesenmaterials aus Titan oder einer Titanlegierung durch thermische Reaktion bei einer Temperatur zwischen 40 und 100°C; und
- das Inkontaktbringen besagten Prothesenmaterials aus Titan oder einer Titanlegierung, auf dem die freien Radikale gebildet worden sind, mit wenigstens einem Monomer, das eine Funktion trägt, die eine radikalische Polymerisation erlaubt, wobei die radikalische Polymerisation besagten Monomers die Bildung eines bioaktiven Polymers erlaubt, wobei besagte radikalische Polymerisation in Abwesenheit von Sauerstoff durchgeführt wird.

**2.** Verfahren gemäß Anspruch 1, wobei die aktiven Donorspezies freier Radikale an der Oberfläche des Prothesenmaterials durch Oxidation mit einer Säure/$H_2O_2$-Mischung erzeugt werden.

**3.** Verfahren gemäß Anspruch 2, wobei das Prothesenmaterial eine Titanlegierung, insbesondere eine auf Nickel, Vanadium, Aluminium, Zirconium und/oder Molybdän basierende Legierung, ist.

**4.** Verfahren gemäß einem der vorhergehenden Ansprüche, wobei die freien Radikale durch thermische Reaktion bei einer Temperatur von 70°C erhalten werden.

5. Verfahren gemäß einem der vorhergehenden Ansprüche, wobei die freien Radikale vor, vorzugsweise während oder nach dem Schritt des Inkontaktbringens der Monomere mit besagtem Material erhalten werden.

6. Verfahren gemäß einem der vorhergehenden Ansprüche, wobei das Monomer wenigstens eine Doppel- oder Dreifachbindung aufweist, insbesondere das Monomer eine Vinylgruppe trägt.

7. Verfahren gemäß einem der vorhergehenden Ansprüche, wobei wenigstens ein Monomer eine Carboxyl- oder Sulfonatfunktion trägt.

8. Verfahren gemäß Anspruch 6, wobei das Monomer Acrylsäure, Methacrylsäure, Methylmethacrylat (MMA), N-(Natriumphenylsulfonat)-acrylamid (NaAS), N-(Natriumphenylsulfonat)-methacrylamid (NaMS), Natriumstyrolsulfonat (NaSS), Ethylenglycolmethacrylatphosphat, Methacryloyl-diisopropyliden, Monomere, die Zuckergruppen wie zum Beispiel Glucose, Glucofuranose, Saccharose, Polyose, Fructose tragen, oder eine beliebige Mischung dieser Verbindungen ist.

9. Verfahren gemäß Anspruch 8, wobei das Monomer Natriumstyrolsulfonat (NaSS) ist.

10. Verfahren gemäß einem der vorhergehenden Ansprüche, wobei besagte Radikalreaktion in Abwesenheit von Sauerstoff in wenigstens ungefähr 30 Minuten, vorzugsweise zwischen 1 und 48 Stunden, vorzugsweise in ungefähr 15 Stunden, durchgeführt wird.

11. Verfahren gemäß einem der vorhergehenden Ansprüche zur Implantation von PolyNaSS auf Titan, umfassend:

- die Oxidation des Titans, um an der Oberfläche besagten Titans aktive Donorspezies freier Radikale zu erzeugen;
- das Inkontaktbringen des Titans, auf dem aktive Spezies erzeugt worden sind, mit NaSS bei einer Temperatur zwischen 40 und 100°C für wenigstens 8 Stunden in Abwesenheit von Sauerstoff.

12. Verfahren gemäß Anspruch 11, umfassend

- die Oxidation des Titans, um an der Oberfläche besagten Titans aktive Donorspezies freier Radikale zu erzeugen;
- das Inkontaktbringen des Titans, auf dem aktive Spezies erzeugt worden sind, mit NaSS bei einer Temperatur von 70°C für 15 Stunden in Abwesenheit von Sauerstoff.

13. Verfahren gemäß einem der vorhergehenden Ansprüche, wobei das Prothesenmaterial vor Bildung der aktiven Spezies an seiner Oberfläche, insbesondere mit Schleifpapier, vorzugsweise mit unterschiedlichen Schleifpapieren mit abnehmender Korngröße bis zu P1200, insbesondere aufeinanderfolgenden Schleifpapieren mit Korngröße P800, P1000 und P1200, poliert wird.

14. Verfahren gemäß einem der vorhergehenden Ansprüche, wobei die Säure/$H_2O_2$-Mischung eine sequentielle Mischung ist, umfassend Inkontaktbringen des Prothesenmaterials mit einer Säurelösung und anschließend Inkontaktbringen mit $H_2O_2$.

15. Verfahren gemäß einem der vorhergehenden Ansprüche, wobei die Säure FluorwasserstoffSäure, Chlorwasserstoff-Säure oder $H_2SO_4$, vorzugsweise $H_2SO_4$, ist.

16. Verfahren gemäß Anspruch 15, wobei das Prothesenmaterial Titan oder eine seiner Legierungen ist, das mit einer $H_2SO_4$-Säurelösung 1 Minute lang, dann mit einer $H_2O_2$-Lösung 2 Minuten lang in Kontakt gebracht wird, wobei das $H_2SO_4/H_2O_2$-Verhältnis vorzugsweise 50/50 (v/v) beträgt.

17. Verfahren gemäß einem der vorhergehenden Ansprüche 2 bis 16, wobei Metallsalze, insbesondere Metallsalze, die aus Eisen-, Nickel-, Chrom- und Kupfersalzen ausgewählt sind, der Säure/$H_2O_2$-Mischung zugegeben werden, wobei die Metallsalze insbesondere mit einer Konzentration zwischen $10^{-4}$ mol/l bis 2 mol/l zugegeben werden.

18. Verfahren gemäß Anspruch 17, wobei das Metallsalz Eisensulfat ist.

19. Prothesenmaterial aus Titan oder einer Titanlegierung, in dem ein bioaktives Polymer implantiert ist, das mit dem

folgenden Verfahren nach einem der Ansprüche 1 bis 18 erhalten werden kann.

**20.** Verwendung von Prothesenmaterial aus Titan oder einer Titanlegierung gemäß Anspruch 19 für die Herstellung eines prothetischen Implantats, insbesondere für die Herstellung einer Gelenkprothese oder einer Zahnprothese.

**21.** Prothetisches Implantat, **dadurch gekennzeichnet, dass** es aus einem Prothesenmaterial aus Titan oder einer Titanlegierung gemäß Anspruch 19 hergestellt ist.

## Claims

**1.** A method for grafting bioactive polymers onto a titanium or titanium alloy prosthetic material, comprising the following steps:

- generating free-radical-donating active species at the surface of the titanium or titanium alloy prosthetic material by oxidizing said titanium or titanium alloy prosthetic material;
- generating free radicals at the surface of the titanium or titanium alloy prosthetic material by thermal reaction at a temperature of between 40 and 100°C; and
- contacting said titanium or titanium alloy prosthetic material onto which free radicals have been generated with at least one monomer bearing a function that allows radical polymerization, the radical polymerization of said monomer allowing the formation of a bioactive polymer, said polymerization being carried out in the absence of oxygen.

**2.** The method according to claim 1, in which the free-radical-donating active species are generated at the surface of the prosthetic material by oxidation with an acid/$H_2O_2$ mixture.

**3.** The method according to claim 2, in which the prosthetic material is an alloy of titanium, in particular a nickel, vanadium, aluminum and/or molybdenum alloy.

**4.** The method according to any one of the preceding claims, in which the free radicals are obtained by thermal reaction at 70°C.

**5.** The method according to any one of the preceding claims, in which the free radicals are obtained before, preferably during or after the step of contacting the monomers with said material.

**6.** The method according to any one of the preceding claims, in which the monomer bears at least one double or triple bond, in particular in which the monomer bears a vinyl group.

**7.** The method according to any one of the preceding claims, in which at least one monomer bears a carboxylate or sulfonate function.

**8.** The method according to claim 6, in which the monomer is acrylic acid, methacrylic acid, methyl methacrylate (MMA), N-(sodium phenylsulfonate) acrylamide (NaAS), N-(sodium phenylsulfonate) methacrylamide (NaMS), sodium styrenesulfonate (NaSS), ethylene glycol methacrylatemethacrylate phosphate, methacryloyldiisopropylidene, monomers bearing oside groups such as glucose, glucofuranose, sucrose, polyose or fructose, or any mixture of these compounds.

**9.** The method according to claim 8, in which the monomer is sodium styrene sulfonate (NaSS) .

**10.** The method according to any one of the preceding claims, wherein said radical reaction is carried out in the absence of oxygen, during at least about 30 minutes, preferably during between 1 and 48 hours, preferably during about 15 hours.

**11.** The method according to any one of the preceding claims, for grafting polyNaSS onto titanium, comprising:

- titanium oxidation to generate free-radical-donating active species at the surface of said titanium;
- contacting the titanium onto which active species have been generated with NaSS at a temperature comprised between 40 and 100°C, during at least 8 hours, in the absence of oxygen.

**12.** The method according to claim 11, comprising:

- oxidizing the titanium to generate free-radical-donating active species at the surface of said titanium;
- contacting the titanium onto which active species have been generated with NaSS at a temperature of 70°C, during 15 hours, in the absence of oxygen.

**13.** The method according to any one of the preceding claims, in which the prosthetic material is polished before formation of the active species on its surface, in particular with abrasive paper, preferably with different abrasive papers of granulometries decreasing down to P1200, in particular successive abrasive papers of granulometry P800, P1000 and P1200.

**14.** The method according to any one of the preceding claims, in which the acid/$H_2O_2$ mixture is a sequential mixture comprising the placing in contact of the prosthetic material with an acid solution, followed by placing it in contact with $H_2O_2$.

**15.** The method according to any one of the preceding claims, in which the acid is hydrofluoric acid, hydrochloric acid or $H_2SO_4$, preferably $H_2SO_4$.

**16.** The method according to claim 15, in which the prosthetic material is titanium or an alloy thereof, which is placed in contact with an acidic $H_2SO_4$ solution for 1 minute and then in contact with an $H_2O_2$ solution for 2 minutes, the $H_2SO_4/H_2O_2$ ratio preferably being 50/50 (v/v).

**17.** The method according to any one of claims 2 to 16, in which metal salts, in particular metal salts selected from iron, nickel, chromium and copper salts, are added to the acid/$H_2O_2$ mixture, the metal salts being more particularly added at a concentration ranging from $10^{-4}$ mol/l to 2 mol/l.

**18.** The method according to claim 17, in which the metal salt is iron sulfate.

**19.** A titanium or titanium alloy prosthetic material onto which is grafted a bioactive polymer obtainable according to the method of any one of claims 1 to 18.

**20.** Use of a titanium or titanium alloy prosthetic material according to claim 19, for the manufacture of a prosthetic implant, in particular for the manufacture of a joint prosthesis or a dental prosthesis.

**21.** A prosthetic implant, **characterized in that** it is manufactured from a titanium or titanium alloy prosthetic material according to claim 19.

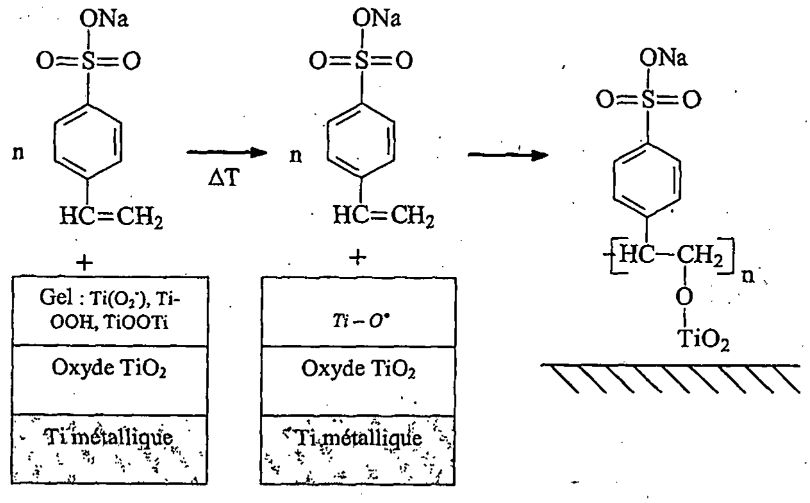
ΔT
Gel : Ti(O2·), Ti-OOH, TiOOTi
Oxyde TiO2
Ti métallique
Ti - O•
Oxyde TiO2
Ti métallique

**Figure 1**

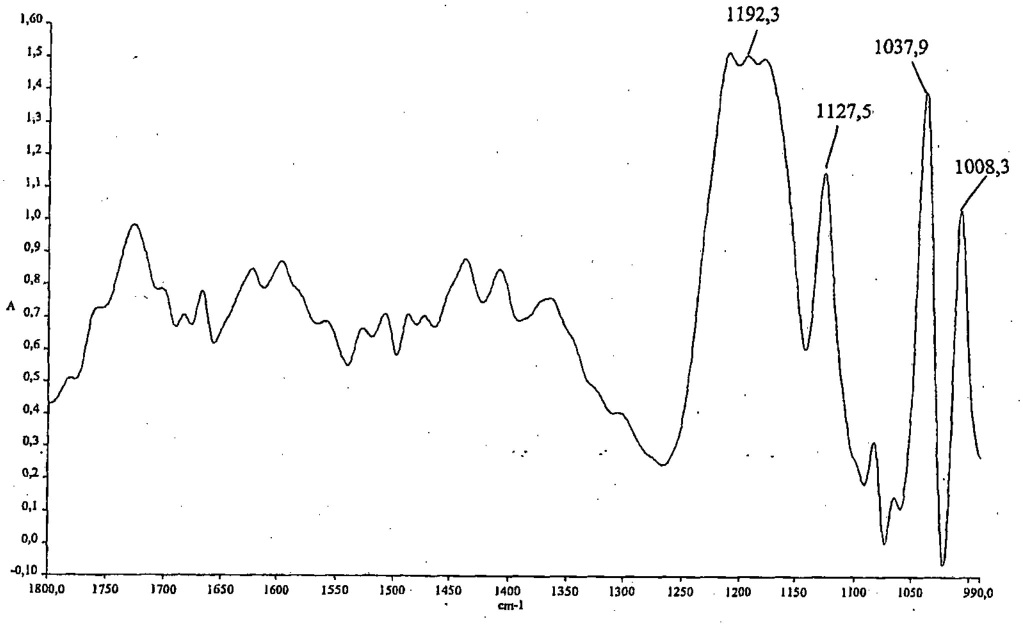
1192,3
1037,9
1127,5
1008,3
1,60
1,5
1,4
1,3
1,2
1,1
1,0
0,9
0,8
A
0,7
0,6
0,5
0,4
0,3
0,2
0,1
0,0
-0,10
1800,0
1750
1700
1650
1600
1550
1500
1450
1400
1350
1300
1250
1200
1150
1100
1050
990,0
cm-1

Figure 2

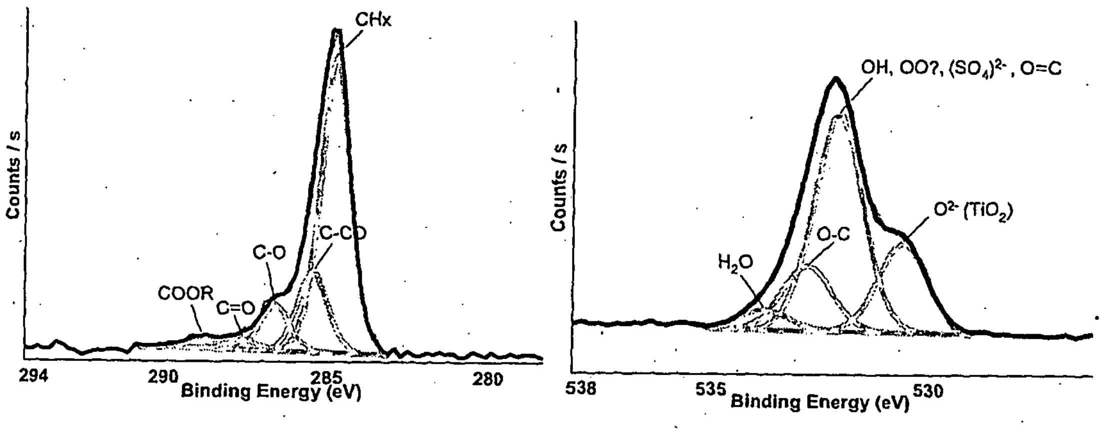


Figure 3A

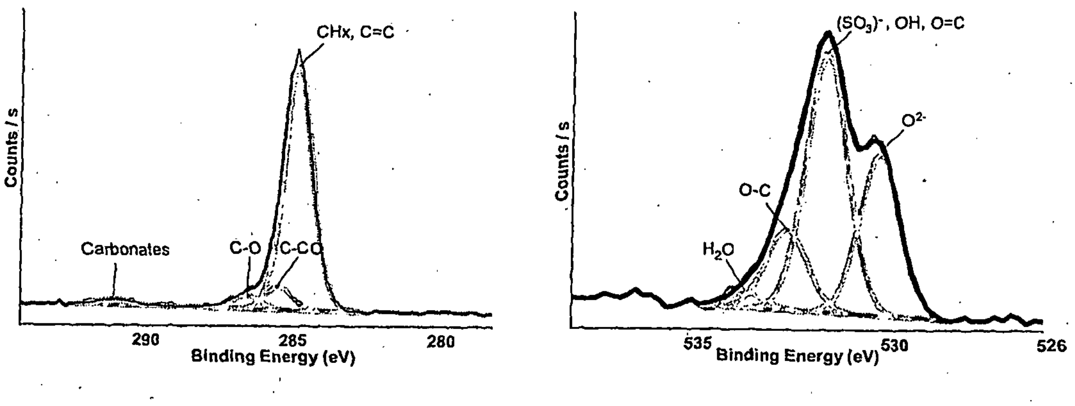


Figure 3B

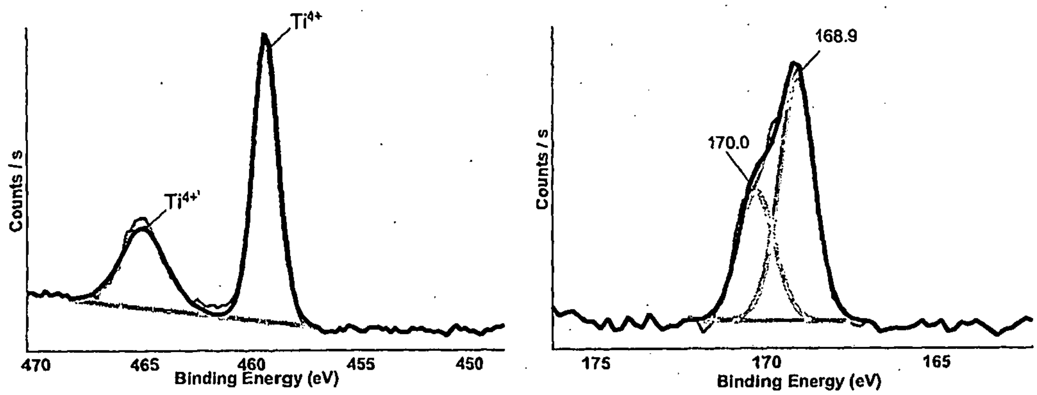


Figure 3C

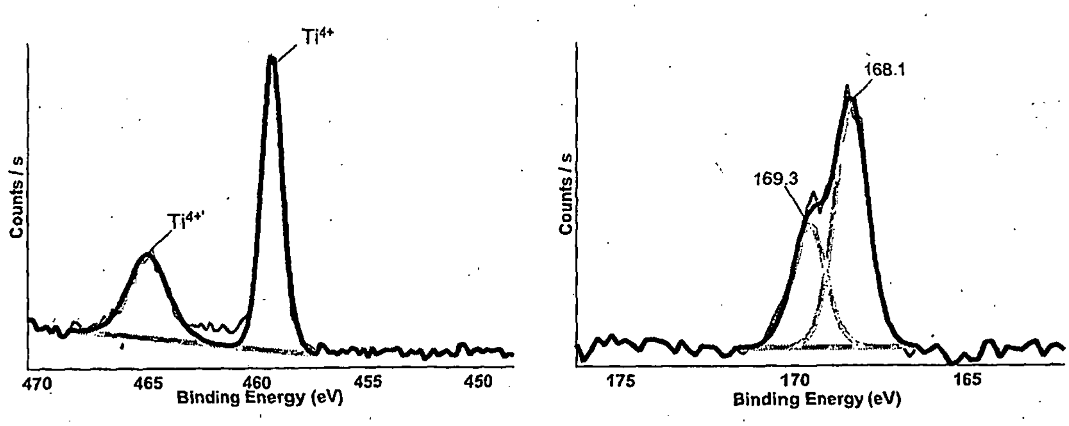


Figure 3D

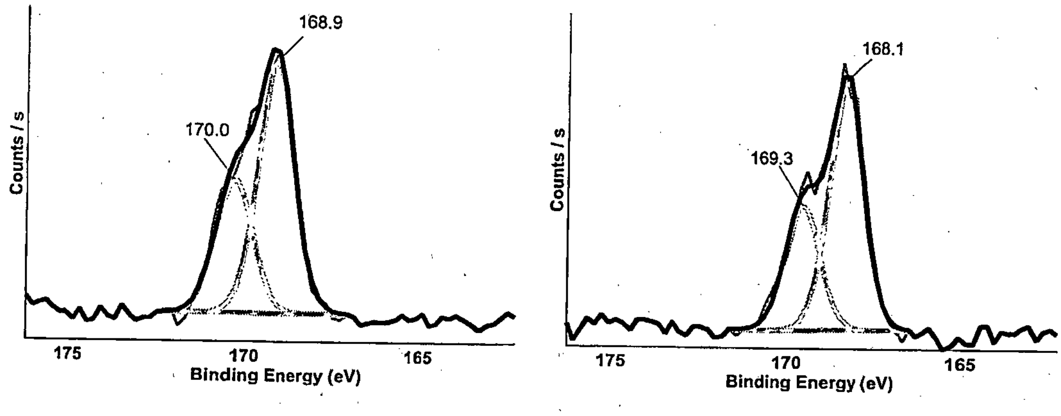
168.9
170.0
Counts / s
175
170
165
Binding Energy (eV)
168.1
169.3
Counts / s
175
170
165
Binding Energy (eV)

**Figure 3E**

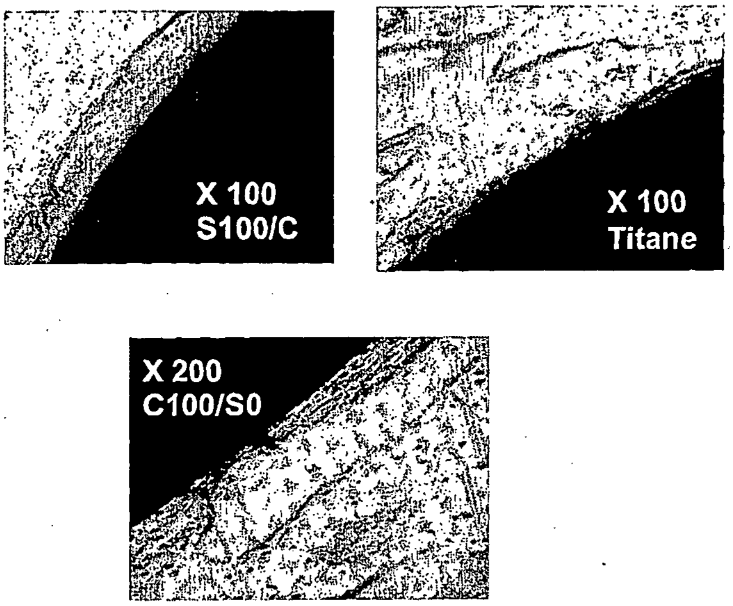


Figure 4